Europäisches Patentamt

European Patent Office

Office européen des brevets

(19)

(11) Veröffentlichungsnummer: **0 296 389 B1**

(12) **EUROPÄISCHE PATENTSCHRIFT**

(45) Veröffentlichungstag der Patentschrift: **06.11.91**

(21) Anmeldenummer: **88108767.0**

(22) Anmeldetag: **01.06.88**

(51) Int. Cl.⁵: **A61K 31/41,** C07D 293/12, A61K 9/14

(54) **Arzneizubereitungen mit mikronisierten EBSELEN-Kristallen.**

(30) Priorität: **20.06.87 DE 3720493**

(43) Veröffentlichungstag der Anmeldung:
**28.12.88 Patentblatt 88/52**

(45) Bekanntmachung des Hinweises auf die
Patenterteilung:
**06.11.91 Patentblatt 91/45**

(84) Benannte Vertragsstaaten:
**AT BE CH DE ES FR GB GR IT LI LU NL SE**

(56) Entgegenhaltungen:
**EP-A- 0 044 971**
**EP-A- 0 249 736**

(73) Patentinhaber: **A. Nattermann & Cie. GmbH**
**Nattermannallee 1**
**W-5000 Köln 30(DE)**

(72) Erfinder: **Römer, Axel, Dr.**
**Heinrich Imig-Str. 16**
**W-5030 Hürth-Gleuel(DE)**
Erfinder: **Seidel, Jürgen, Dr.**
**Venloer Str. 67**
**W-5024 Pulheim(DE)**

(74) Vertreter: **Redies, Bernd, Dr. rer. nat.**
**COHAUSZ & FLORACK Patentanwaltsbüro**
**Schumannstrasse 97 Postfach 14 01 20**
**W-4000 Düsseldorf 1(DE)**

## Beschreibung

Die vorliegende Erfindung betrifft feste Arzneizubereitungen von 2-Phenyl-1,2-benzisoselenazol-3(2H)-on (EBSELEN), die eine hohe Bioverfügbarkeit des Wirkstoffs ergeben und andere wertvolle pharmakologische Eigenschaften haben, ihre Verwendung sowie Verfahren zur Herstellung dieser festen Arzneizubereitungen.

EBSELEN ist eine bekannte Substanz (DE-PS 3027073) und kann nach dem Verfahren von R. Weber und M. Renson, Bulletin de la Soc. Chim. De France 1976 (7/8), 1124-1126, durch Umsetzung von 2-Methylseleno-N-phenyl-benzamid mit Phosphorpentachlorid und anschließender Hydrolyse hergestellt werden.

EBSELEN-Präparate können zur Behandlung zahlreicher Krankheiten verwendet werden, wie z.B. zur Prophylaxe und Therapie von Infektionskrankheiten, zur Therapy von malignen Tumoren, zur Stimulierung des Immunsystems oder bei Selen-Mangelkrankheiten. Hervorzuheben sind antiarteriosklerotische und entzündungshemmende Eigenschaften zur Therapie von rheumatischen Krankheiten. Darüberhinaus eignet sich EBSELEN zur Behandlung von Krankheiten, die durch eine Zellschädigung aufgrund vermehrter Bildung von aktiven Sauerstoffmetaboliten hervorgerufen werden, wie z.B. Leberschäden, Herzinfarkt, Psoriasis und Strahlenschäden.

EP-A-0 249 736, welche nach Artikel 54(3) EPÜ zu berücksichtigen ist, beschreibt Salben, welche EBSELEN mit einer Partikelgröße von weniger als 5 $\mu$m enthalten.

Dem breiten Wirkungsspektrum entgegen steht die geringe Wasserlöslichkeit des EBSELENS und die dadurch ungünstig beeinflußte Bioverfügbarkeit.

Es ist Aufgabe der vorliegenden Erfindung, feste Arzneizubereitungen des EBSELENS zu schaffen, die eine verbesserte Bioverfügbarkeit besitzen.

Die festen Arzneizubereitungen von EBSELEN gemäß der vorliegenden Erfindung liegen in Form von Tabletten, Pillen, Dragees, Kapseln, Suppositorien oder Granulate vor und sind dadurch charakterisiert, daß sie EBSELEN in mikronisierter Form mit einem durchschnittlichen Teilchendurchmesser der EBSELEN-Teilchen von weniger als 10 $\mu$m, vorzugsweise zwischen 10 und 0,5 $\mu$m.

Durch die Bioverfügbarkeit wird vor allem die nach Applikation einer Arzneiform in den Blutkreislauf gelangte Wirkstoffmenge charaktersiert. Zur Prüfung der Bioverfügbarkeit werden im allgemeinen Blutspiegelkurven verwendet. Voraussetzung hierfür ist, daß der Arzneistoff oder relevante Metaboliten im Blut mit chemisch-analytischen Verfahren erfaßbar ist und daß der Blutspiegel und die therapeutische Wirksamkeit des Arzneistoffs in Relation stehen.

Verglichen werden die Flächen unter den Blut(plasma)konzentrations-Zeit-Kurven (AUC = are under curve). Die Fläche unter der Kurve entspricht der Arzneistoffmenge, welche die systemische Zirkulation erreicht, in Abhängigkeit vom Verteilungsvolumen und der Eliminationsgeschwindigkeitskonstante (Metabolismus und Ausscheidung).

Zur Charakterisierung der Bioverfügbarkeit werden die folgenden Parameter herangezogen:
die Fläche unter der Blutspiegelkurve (AUC),
die Höhe des Blutspiegelmaximums $C_{max}$,
die Zeit bis zum Erreichen des Blutspiegelmaximums $T_{max}$.

Bei Blutspiegeluntersuchungen nach gleichen Dosierungen an Minipigs wurde gefunden, daß mikronisiertes EBSELEN eine überraschende Zunahme der Bioverfügbarkeit bewirkt.

Das bei der Synthese anfallende und bisher verwendete kristalline EBSELEN mit einer mittleren Teilchengröße von 70 $\mu$m wurde verglichen mit mikronisiertem EBSELEN mit einer mittleren Teilchengröße von weniger als 10 $\mu$m, insbesondere 2 $\mu$m.

Das EBSELEN wurde in Hartgelatinekapseln in mikronisierter (2 $\mu$m) und kristalliner (70 $\mu$m) Form in einer Dosierung von 50 mg/kg Körpergewicht an fastende Minipigs verabreicht.

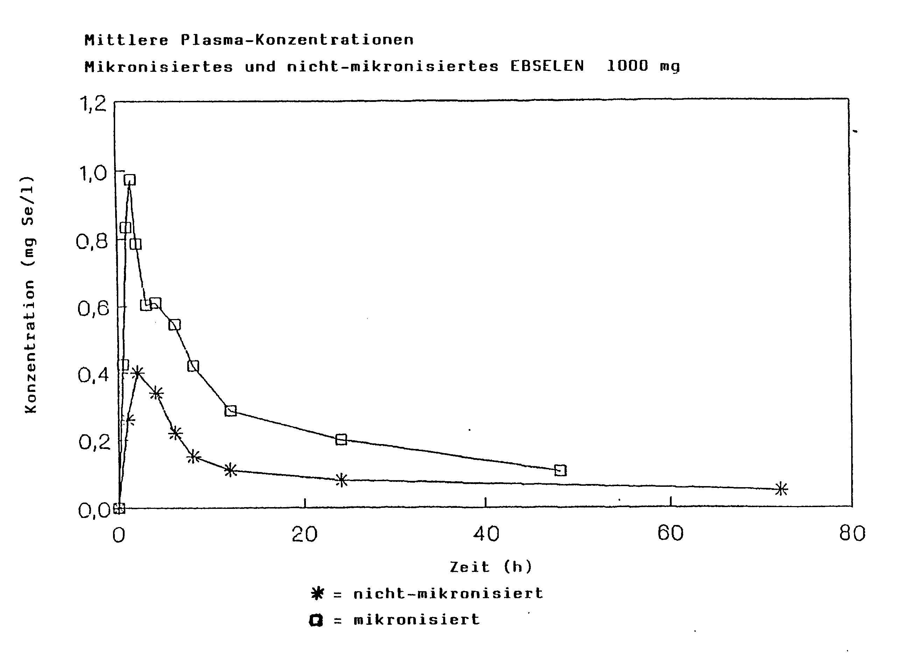

Die auf diese Weise erhaltenen Plasma-Konzentrationskurven in Abhängigkeit von der Zeit sind in Abbildung 1 dargestellt und zweigen bei ähnlichem Verlauf sehr deutliche Unterschiede in ihren $C_{max}$-Werten und folglich auch in den AUC-Werten.

Mit der mikronisierten Form wurden zwischen der 1. bis 4. Stunde nach der Einnahme Plasmaspiegel von 4,5 bis 5,5 mg Se/l erreicht. Bei dem 70 $\mu$m-EBSELEN war die Plasmakonzentration deutlich niedriger und bewegte sich bei ähnlichem $T_{max}$ zwischen 1,5 - 2,5 mg Se/l.

Nach 48 Stunden erreichten die Plasmaspiegel in beiden Fällen den Ausgangswert.

Die mittleren AUC-Werte (0 - 48 h) der beiden Formulierungen betrugen
41 mg x h/l für die 70 $\mu$m-Form
61 mg x h/l für die 2 $\mu$m-Form.

Die mittlere kumulative Ausscheidung über den Harn - in % der Dosierung - betrug über einen Zeitraum von 48 Stunden 28 für die 70 μm-Form und 59 für die 2 μm-Form.

Aus diesen Werten wird ersichtlich, daß die neue mikronisierte Form des EBSELENS erhebliche Vorteile gegenüber der 70 μm-Form bietet.

Der mittlere Plasmaspiegel ist um den Faktor 2 erhöht, ebenso die Ausscheidung über den Urin, der AUC-Wert ist um ungefähr 50% größer.

Die neue mikronisierte Form des EBSELEN zeigt eine erhöhte Bioverfügbarkeit und kann mit größeren Erfolgsaussichten zur Behandlung zahlreicher Krankheiten verwendet werden, wie z.B. zur Prophylaxe und Therapie von Infektionskrankheiten, zur Stimulierung des Immunsystems oder bei Selenmangelkrankheiten.

Die neue mikronisierte Form zeichnet sich besonders durch antiarteriosklerotische und entzündungshemmende Eigenschaften aus. Sie eignet sich daher besonders zur Therapie von rheumatischen Krankheiten, wie z.B. Arthrosen oder chronischer Polyarthritis, zur Lebertherapie, zur Behandlung von Hautkrankheiten wie Psoriasis. Die neuen Formulierungen zeichnen sich durch eine sehr gute Verträglichkeit aus, da sie untoxisch sind und im Gegensatz zu bekannten entzündungshemmenden Therapeutika keinerlei Ulcusbildung oder gastrointestinale Irritationen zeigen.

Die vorliegende Erfindung betrifft auch ein Verfahren zur Herstellung von festen Arzneizubereitungen, welche EBSELEN-Kristalle mit einem mittleren Durchmesser von weniger als 10 μm, insbesondere 0,5 bis 10 μm, z.B. 2 μm enthalten.

Die Herstellung der erfindungsgemäß verwendeten EBSELEN-Kristalle mit einem mittleren Durchmesser von 2 μm erfolgt, indem man die aus der Synthese von EBSELEN erhaltenen Kristalle mahlt. Die Mahlung kann z.B. mit Stiftmühlen oder Hammermühlen erfolgen. Durch Variation der Drehzahl der Mühle, der zulaufenden Menge Produkt und/oder der Mahlungsdauer kann EBSELEN der gewünschten Teilchengröße gewonnen werden. Besonders vorteilhaft ist die Mahlung mit Luftstrahlmühlen durchzuführen. Die Herstellung der erfindungsgemäßen Arzneizubereitungen erfolgt nach üblichen Methoden, indem man mikronisierte EBSELEN-Kristalle mit geeigneten Hilfsstoffen mischt oder granuliert und aus den Mischungen oder Granulaten nach üblichen Verfahren feste Arzneizubereitungen herstellt.

Als feste Arzneizubereitungen seien vorzugsweise genannt: Tabletten, Pillen, Dragees und Kapseln.

Die vorliegende Erfindung betrifft ebenfalls pharmazeutische Präparate, welche mikronisiertes EBSELEN enthalten. Bei den erfindungsgemäßen pharmazeutischen Präparaten handelt es sich um solche zu enteralen, wie oralen oder rektalen sowie parenteralen Verabreichung, welche den pharmazeutischen Wirkstoff allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind.

Die Dosierung des Wirkstoffes liegt üblicherweise zwischen 10 und 2000 mg pro Tag, vorzugsweise zwischen 30 und 300 mg pro Tag und kann in einer Dosis oder mehreren Teildosen, vorzugsweise in zwei bis drei Teildosen pro Tag, verabreicht werden.

Die Herstellung der erfindungsgemäßen Arzneizubereitungen wird durch die folgenden Beispiele näher erläutert.

Beispiel 1

| Tablette | |
|---|---|
| Mikronisiertes EBSELEN mit einem durchschnittlichen Teilchendurchmesser von 0,5 μm | 250 mg |
| Lactose | 160 mg |
| Kollidon 25 | 10 mg |
| Maisstärke | 52 mg |
| Talkum | 12 mg |

Die aufgeführten Stoffe werden nach üblichen Verfahren gemischt und gepreßt. Die Preßlinge können gegebenenfalls mit einem Filmüberzug versehen werden.

Beispiel 2

| Kapsel | |
|---|---|
| Mikronisiertes EBSELEN mit einem durchschnittlichen Teilchendurchmesser von 2 $\mu$m | 100 mg |
| Talkum | 10 mg |
| Aerosil 200 | 20 mg |

Die aufgeführten Stoffe werden nach üblichen Verfahren gemischt, granuliert und in Hartgelatinekapseln abgefüllt.

## Patentansprüche
### Patentansprüche für folgende Vertragsstaaten : BE, CH, DE, ES, FR, GB, GR, IT, Li, LU, NL, SE

1. Feste Arzneizubereitungen von 2-Phenyl-1,2-benzisoselenazol-3(2H)-on (EBSELEN) mit hoher Bioverfügbarkeit in Form von Tabletten, Pillen, Dragees, Kapseln, Suppositorien oder Granulaten, dadurch gekennzeichnet, daß sie das 2-Phenyl-1,2-benzisoselenazol-3(2H)-on (EBSELEN) in Form mikronisierter Kristalle mit einem durchschnittlichen Teilchendurchmesser von weniger als 10 $\mu$m haben.

2. Arzneizubereitungen gemäß Anspruch 1, dadurch gekennzeichnet, daß sie EBSELEN-Kristalle mit einem mittleren Durchmesser zwischen 10 und 0,5 $\mu$m enthalten.

3. Verfahren zur Herstellung von festen Arzneizubereitungen gemäß den Ansprüchen 1 oder 2, dadurch gekennzeichnet, daß man aus der Synthese erhaltene EBSELEN-Kristalle durch Mahlung oder Siebung in ein Kristallpulver mit einem mittleren Teilchendurchmesser von weniger als 10 $\mu$m überführt und aus diesen EBSELEN-Kristallen nach üblichen Methoden unter Verwendung von Hilfs- und Trägerstoffen feste Arzneizubereitungen formuliert.

4. Verwendung von EBSELEN-Kristallen zur Herstellung von Arzneizubereitungen nach Anspruch 1 oder 2.

### Patentansprüche für folgenden Vertragsstaat : AT

1. Verfahren zur Herstellung von festen Arzneimittelzubereitungen von 2-Phenyl-1,2-benzisoselenazol-3-(2H)-on (EBSELEN) mit hoher Bioverfügbarkeit in Form von Tabletten, Pillen, Dragees, Kapseln, Suppositorien oder Granulaten, dadurch gekennzeichnet, daß man aus der Synthese erhaltene EBSELEN-Kristalle durch Mahlung oder Siebung in ein Kristallpulver mit einem mittleren Teilchendurchmesser von weniger als 10 $\mu$m überführt und aus diesen EBSELEN-Kristallen nach üblichen Methoden unter Verwendung von Hilfs- und Trägerstoffen feste Arzneimittelzubereitungen formuliert

2. Verfahren gemäß Anspruch 1, dadurch gekennzeichnet, daß sie EBSELEN-Kristalle mit einem mittleren Durchmesser zwischen 10 und 0,5 $\mu$m enthalten.

3. Verwendung von EBSELEN-kristallen zur Herstellung von Arzneimittelzubereitungen nach Anspruch 1 oder 2.

## Claims
### Claims for the following Contracting States : BE, CH, DE, ES, FR, GB, GR, IT, LI, LU, NL, SE

1. Solid 2-phenyl-1,2-benzisoselenazol-3(2H)-one (EBSELEN) pharmaceutical preparations having high bioavailability, in the form of tablets, pills, coated tablets, capsules, suppositories or granules, characterised in that they contain 2-phenyl-1,2-benzisoselenazol-3(2H)-one (EBSELEN) in the form of micronised crystals having an average particle diameter of less than 10 $\mu$m.

2. Pharmaceutical preparations according to Claim 1, characterised in that they contain EBSELEN crystals having an average diameter between 10 and 0.5 $\mu$m

3. Process for the production of solid pharmaceutical preparations according to Claims 1 or 2, characterised in that EBSELEN crystals obtained from synthesis are converted into a crystal powder having an average particle diameter of less than 10 $\mu$m by grinding or sieving and solid pharmaceutical preparations are formulated from these EBSELEN crystals by customary methods using auxiliaries and excipients.

4. Use of EBSELEN crystals for the production of pharmaceutical preparations according to Claim 1 or 2.

**Claims for the following Contracting State : AT**

1. Process for the production of solid 2-phenyl-1,2-benzisoselenazol-3(2H)-one (EBSELEN)pharmaceutical preparations having high bio-availability, in the form of tablets, pills, coated tablets, capsules, suppositories or granules, characterised in that EBSELEN crystals obtained from synthesis are converted into a crystal powder having an average particle diameter of less than 10 μm by grinding or sieving and solid pharmaceutical preparations are formulated from these EBSELEN crystals by customary methods using auxiliaries and excipients.

2. Process according to Claim 1, characterised in that the preparations contain EBSELEN crystals having an average diameter between 10 and 0.5 μm.

3. Use of EBSELEN crystals for the production of pharmaceutical preparations in the process according to Claim 1 or 2.

**Revendications**
**Revendications pour les Etats contractants suivants : BE, CH, DE, ES, FR, GB, GR, IT, LI, LU, NL, SE**

1. Compositions médicamenteuses solides à base de la 2-phényl-1,2-benzoisosélénazole-3(2H)-one (EBSELEN) à haute biodisponibilité à l'état de comprimés, de pilules, de dragées, de capsules, de suppositoires ou de granulés, caractérisées en ce qu'elles contiennent la 2-phényl-1,2-benzoisosélénazole-3(2H)-one (EBSELEN) à l'état de cristaux micronisés à un diamètre de particule moyen inférieur à 10 μm.

2. Compositions médicamenteuses selon la revendication 1, caractérisées en ce qu'elles contiennent des cristaux d'EBSELEN à un diamètre moyen de 10 à 0,5 μm.

3. Procédé de préparation des compositions médicamenteuses solides selon la revendication 1 ou 2, caractérisé en ce que l'on met les cristaux d'EBSELEN, tels qu'obtenus à la synthèse, à l'état de poudre cristalline d'un diamètre de particule moyen inférieur à 10 μm par broyage ou tamisage et on met ces cristaux d'EBSELEN à l'état de compositions médicamenteuses solides par des modes opératoires usuels avec utilisation de produits auxiliaires et véhicules.

4. Utilisation de cristaux d'EBSELEN pour la préparation de compositions médicamenteuses selon la revendication 1 ou 2.

**Revendications pour l'Etat contractant suivant : AT**

1. Procédé de préparation de compositions médicamenteuses solides à base de 2-phényl-1,2-benzoisosélénazole-3(2H)-one (EBSELEN) à haute biodisponibilité à l'état dé comprimés, de pilules, de dragées, de capsules, de suppositoires ou de granules, caractérisé en ce que l'on met les cristaux d'EBSELEN, tels qu'obtenus à la synthèse, à l'état de poudre cristalline à un diamètre de particule moyen inférieur à 10 μm par broyage ou tamisage et on met ces cristaux d'EBSELEN à l'état de compositions médicamenteuses solides par des modes opératoires usuels avec utilisation de produits auxiliaires et véhicules.

2. Procédé selon la revendication 1, caractérisé en ce que les compositions contiennent les cristaux d'EBSELEN à un diamètre moyen de 10 à 0,5 μm.

3. Utilisation de crixtaux d'EBSELEN pour la préparation de compositions médicamenteuses selon la revendication 1 ou 2.

Mittlere Plasma-Konzentrationen

Mikronisiertes und nicht-mikronisiertes EBSELEN   1000 mg

EP 0 296 389 B1